(19) Europäisches Patentamt
European Patent Office
Office européen des brevets

(11) EP 3 851 509 A1

(12) EUROPEAN PATENT APPLICATION

(43) Date of publication:
**21.07.2021 Bulletin 2021/29**

(21) Application number: **20201717.4**

(22) Date of filing: **14.10.2020**

(51) Int Cl.:
***C11D 1/26*** *(2006.01)* ***C11D 1/94*** *(2006.01)*
***C11D 3/00*** *(2006.01)* ***C11D 11/00*** *(2006.01)*
***C11D 17/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **14.01.2020 EP 20151591**

(71) Applicant: **The Procter & Gamble Company**
**Cincinnati, OH 45202 (US)**

(72) Inventors:
- **BRAECKMAN, Karl Ghislain**
  **1853 Strombeek-Bever (BE)**
- **VANDENBERGHE, Frederik Clara P.**
  **1853 Strombeek-Bever (BE)**

(74) Representative: **P&G Patent Belgium UK**
**N.V. Procter & Gamble Services Company S.A.**
**Temselaan 100**
**1853 Strombeek-Bever (BE)**

# (54) LIQUID DETERGENT COMPOSITION

(57) The need for a liquid detergent composition, especially a hand dishwashing liquid detergent which provides a more consistent sudsing experience, regardless of the hardness of the water used to prepare the cleaning liquor, while also providing improved initial suds formation and suds mileage in the presence of greasy soils, especially under hard water conditions, is met by formulating a liquid detergent composition to comprise an anionic surfactant and a co-surfactant, wherein the anionic surfactant comprises an alkyl isosorbide sulphate anionic surfactant.

EP 3 851 509 A1

## Description

### FIELD OF THE INVENTION

The present invention relates to a liquid detergent composition, more particularly to liquid hand dishwashing detergent compositions.

### BACKGROUND OF THE INVENTION

During manual dishwashing in a sink full of water into which a detergent composition has been diluted to form a cleaning liquor, the user typically relies on the level of suds to indicate the remaining cleaning efficacy of the diluted detergent composition. A high suds volume and/or stable, long-lasting suds longevity (*i.e.*, mileage) indicates to the user that sufficient active ingredients (e.g., surfactants) remain, in order to perform the desired cleaning. Poor suds longevity typically leads to the user dosing additional detergent composition even when cleaning efficacy remains.

Anionic surfactants have been used, typically in combination with cosurfactants, especially amphoteric and zwitterionic co-surfactants such as amine oxide and betaines, to provide suds during dishwashing, with alkyl sulphate and alkyl alkoxy sulphates being found to be particularly effective at providing improved sudsing in addition to the desired cleaning.

The suds volume and longevity are significantly affected by the level of the hardness of the water used. As such, the satisfaction of the user can often depend not on the efficacy of the detergent composition itself, but on the hardness of the water used. Moreover, formulating the detergent composition to provide good suds in hard water conditions can result in excessive sudsing when soft water is used.

Hence, there remains a need for a liquid detergent composition, especially a hand dishwashing liquid detergent which provides a more consistent sudsing experience, regardless of the hardness of the water used to prepare the cleaning liquor. Beyond reduced sensitivity to water hardness there remains a need to still further boost initial suds formation especially when applied undiluted on a cleaning implement such as a sponge, as well as suds mileage when diluted in a cleaning liquor in presence of greasy soils especially under hard water conditions.

Lavergne, A., et al., 2011, "Synthesis and foaming properties of new anionic surfactants based on a renewable building block: Sodium dodecyl isosorbide sulfates", J. Colloid Interface Sci, 2011, vol. 360(2), pp645-653, describes two isosorbide-based anionic surfactants, and their method of synthesis, as well as their amphiphilic properties. EP2270017B1 describes isosorbide derivatives and their use to prepare cleansers, detergents, personal care compositions, or cosmetic compositions. EP2758404B1 relates to isosorbide derivatives for use in producing cosmetic compositions. EP2343301B1 relates to anionic isosorbide derivatives and their use to prepare cleansers, detergents, personal care compositions, or cosmetic compositions. EP2336281A1 relates to isosorbide phosphates and their use in the preparation of cleaners, detergents, personal care, cosmetic or pharmaceutical compositions, preferably in cleaners, personal care and cosmetic compositions. EP3165593A1 relates to a liquid detergent composition having a pH of from 7.1 to less than 8.9 as measured at 10% solution in distilled water at 20°C wherein the composition comprises a surfactant system, the surfactant system comprising an anionic surfactant and a primary co-surfactant selected from the group consisting of amphoteric surfactant, zwitteronic surfactant and mixtures thereof wherein the anionic surfactant and the primary co-surfactant are in a weight ratio of from less than 10:1 to more than 2.5:1 and wherein the composition further comprises a specific cyclic amine. WO2013/041388A1 relates to specific isosorbide derivatives which are suitable for producing cosmetic compositions.

### SUMMARY OF THE INVENTION

The present invention relates to a liquid detergent composition, preferably a liquid hand dishwashing detergent composition, comprising from 5% to 50% by weight of the total composition of a surfactant system, wherein the surfactant system comprises an anionic surfactant at a level of from 60% to 90% of the surfactant system, and a co-surfactant, wherein: the anionic surfactant comprises from 10% to 100% of the anionic surfactant an alkyl isosorbide sulphate anionic surfactant wherein the alkyl isosorbide sulphate anionic surfactant has a structure according to formula (I) as described herein, or an isomer thereof; and the co-surfactant is selected from the group consisting of an amphoteric surfactant, a zwitterionic surfactant and mixtures thereof, wherein the pH of the composition is at least 7.0 as measured at 10% dilution in distilled water at 20°C.

The present invention further relates to a method of manually washing dishware comprising the steps of: delivering the composition of the invention to a volume of water to form a wash solution and immersing the dishware in the solution.

DETAILED DESCRIPTION OF THE INVENTION

The liquid detergent compositions of the present invention provide a more consistent sudsing experience, regardless of the hardness of the water used to make the wash solution.

The compositions of the present invention also provide a good sudsing profile, including high initial suds volume generation and sustained suds stabilization through the dishwashing process, even when in presence of greasy and/or particulate soils, as well as good grease removal, in particular good removal of uncooked grease and particulate soils.

Definitions

As used herein, articles such as "a" and "an" when used in a claim, are understood to mean one or more of what is claimed or described.

The term "comprising" as used herein means that steps and ingredients other than those specifically mentioned can be added. This term encompasses the terms "consisting of' and "consisting essentially of." The compositions of the present invention can comprise, consist of, and consist essentially of the essential elements and limitations of the invention described herein, as well as any of the additional or optional ingredients, components, steps, or limitations described herein.

The term "dishware" as used herein includes cookware and tableware made from, by non-limiting examples, ceramic, china, metal, glass, plastic (e.g., polyethylene, polypropylene, polystyrene, etc.) and wood.

The term "grease" or "greasy" as used herein means materials comprising at least in part (*i.e.*, at least 0.5 wt% by weight of the grease) saturated and unsaturated fats and oils, preferably oils and fats derived from animal sources such as beef, pig and/or chicken.

The terms "include", "includes" and "including" are meant to be non-limiting.

The term "particulate soils" as used herein means inorganic and especially organic, solid soil particles, especially food particles, such as for non-limiting examples: finely divided elemental carbon, baked grease particle, and meat particles.

The term "sudsing profile" as used herein refers to the properties of a detergent composition relating to suds character during the dishwashing process. The term "sudsing profile" of a detergent composition includes suds volume generated upon dissolving and agitation, typically manual agitation, of the detergent composition in the aqueous washing solution, and the retention of the suds during the dishwashing process. Preferably, hand dishwashing detergent compositions characterized as having "good sudsing profile" tend to have high suds volume and/or sustained suds volume, particularly during a substantial portion of or for the entire manual dishwashing process. This is important as the consumer uses high suds as an indicator that sufficient detergent composition has been dosed. Moreover, the consumer also uses the sustained suds volume as an indicator that sufficient active cleaning ingredients (*e.g.*, surfactants) are present, even towards the end of the dishwashing process. The consumer usually renews the washing solution when the sudsing subsides. Thus, a low sudsing detergent composition will tend to be replaced by the consumer more frequently than is necessary because of the low sudsing level.

It is understood that the test methods that are disclosed in the Test Methods Section of the present application must be used to determine the respective values of the parameters of Applicants' inventions as described and claimed herein.

In all embodiments of the present invention, all percentages are by weight of the total composition, as evident by the context, unless specifically stated otherwise. All ratios are weight ratios, unless specifically stated otherwise, and all measurements are made at 25°C, unless otherwise designated.

Liquid detergent composition

The detergent composition is preferably a hand dishwashing detergent composition in liquid form. The liquid detergent composition is preferably an aqueous detergent composition. As such, the composition can comprise from 50% to 85%, preferably from 50% to 75%, by weight of the total composition of water.

The pH of the composition is at least 7.0, preferably from about 7.0 to about 12.0, or more preferably from about 7.5 to about 10.0, as measured at 10% dilution in distilled water at 20°C. The pH of the composition can be adjusted using pH modifying ingredients known in the art.

The composition of the present invention can be Newtonian or non-Newtonian, preferably Newtonian. Preferably, the composition has a viscosity of from 10 mPa·s to 10,000 mPa·s, preferably from 100 mPa·s to 5,000 mPa·s, more preferably from 300 mPa·s to 2,000 mPa·s, or most preferably from 500 mPa·s to 1,500 mPa·s, alternatively combinations thereof. The viscosity is measured with a Brookfield RT Viscometer using spindle 21 at 20 RPM at 25°C.

Surfactant System

The detergent composition can comprise from 5% to 50%, preferably from 8% to 45%, most preferably from 15% to 40%, by weight of the total composition of a surfactant system. The surfactant system comprises anionic surfactant and a co-surfactant which is selected from the group consisting of an amphoteric surfactant, a zwitterionic surfactant and mixtures thereof.

In order to improve surfactant packing after dilution and hence improve robustness against hardness variations and suds mileage, the surfactant system can comprise the anionic surfactant and co-surfactant in a weight ratio of from 1:1 to 9:1, preferably 2:1 to 5:1, more preferably 2.5:1 to 4:1.

Anionic surfactant:

The composition comprises from 60% to 90%, preferably from 65% to 85%, more preferably from 70% to 80% by weight of the surfactant system of the anionic surfactant. The anionic surfactant comprises from 10% to 100%, preferably from 20% to 80%, more preferably from 25% to 35% by weight of the anionic surfactant of an alkyl isosorbide sulphate anionic surfactant.

Suitable alkyl isosorbide sulphate anionic surfactants have a structure according to formula (I), or an isomer thereof:

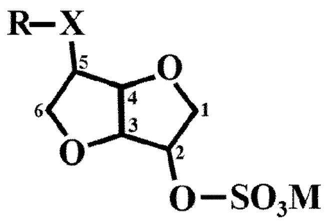

(I)

wherein:

R has an average alkyl chain length of from 8 to 18, preferably from 10 to 14, more preferably from 12 to 14, most preferably from 12 to 13 carbon atoms; R is preferably linear, and more preferably naturally derived, such as from coconut oil, palm kernel oil, and mixtures thereof, most preferably coconut oil;

X is a linking group, preferably a linking group selected from an oxygen atom (-O-), an ester group (-(C=O)-O-), or a mixture thereof, more preferably an oxygen atom;

M is a cation, preferably a counter-ion selected from H, an alkali metal atom, an alkali earth metal ion, an ammonium, an alkylammonium ion, or a mixture thereof, preferably M is an alkali metal ion, more preferably M is Sodium,

wherein the alkyl isosorbide sulphate anionic surfactant comprises at least 70% by weight of the alkyl isosorbide sulphate anionic surfactant of the 5-isomer alkyl isosorbide sulphate anionic surfactant, wherein the 5-isomer alkyl isosorbide sulphate anionic surfactant has the alkyl chain R bound via the linking group X to the 5 position in the isosorbide group.

Such surfactants can be derived from isosorbide (or 1,4: 3,6-dianhydrosorbitol) which is the anhydride of sorbitol. Such alkyl isosorbide sulphate anionic surfactants can be produced as described in Lavergne, A., et al., 2011, "Synthesis and foaming properties of new anionic surfactants based on a renewable building block: Sodium dodecyl isosorbide sulfates", J. Colloid Interface Sci, 2011, vol. 360(2), pp645-653, or in EP2270017B1, such as in example 1 and 2 of EP2270017B1.

The alkyl isosorbide sulphate anionic surfactants of formula I can comprise one of two isomers, or a blend of the two isomers. The 5-isomer alkyl isosorbide sulphate anionic surfactant has the alkyl chain R bound via the linking group X to the 5 position in the isosorbide group. Such 5-isomer alkyl isosorbide sulphate surfactants include, for example, 5-O-dodecyl isosorbide sulphate. In this case the sulphate group is attached to the 2 position in the isosorbide group. Alternatively, the 2-isomer alkyl isosorbide sulphate anionic surfactant, such as 2-O-dodecyl isosorbide sulphate can be used, in which the alkyl chain R is bound via the linking group X to the 2 position in the isosorbide group and the sulphate group being bound to the 5 position in the isosorbide group instead.

The 5-isomer alkyl isosorbide sulfate is preferred since it is the most soluble and most efficient of the two isomers. This is because the 5-isomer alkyl isosorbide sulphate surfactant has a lower critical micelle concentration (cmc) leading to comparable foaming properties at a lower concentration. As such, the alkyl isosorbide sulphate anionic surfactant comprises at least 70%, preferably at least 80%, more preferably at least 85% by weight of the alkyl isosorbide sulphate anionic surfactant of the 5-isomer alkyl isosorbide sulphate anionic surfactant, wherein the 5-isomer alkyl

isosorbide sulphate anionic surfactant has the alkyl chain R bound via the linking group X to the 5 position in the isosorbide group.

The alkyl isosorbide sulfate anionic surfactant is preferably non-ethoxylated, more preferably non-alkoxylated. Without wishing to be bound by theory, it is believed that alkoxyated materials are inferior in sudsing performance in comparison to the non-alkoxylated isosorbide moiety, as alkoxylation produces a distribution of materials with different alkoxylation degree, among which a significant proportion of higher alkoxylates with inferior sudsing performance are present.

The anionic surfactant can further comprise an alkyl sulphate surfactant. If present, the alkyl sulphate surfactant can have a weight average degree of alkoxylation of less than 3, preferably less than 2.0, more preferably less than 1.0, and most preferably wherein the alkyl sulphate surfactant is not alkoxylated.

The average degree of alkoxylation is the mol average degree of alkoxylation (*i.e.*, mol average alkoxylation degree) of all the alkyl sulphate anionic surfactant. Hence, when calculating the mol average alkoxylation degree, the mols of non-alkoxylated sulphate anionic surfactant are included:

$$\text{Mol average alkoxylation degree} = (x1 * \text{alkoxylation degree of surfactant 1} + x2 * \text{alkoxylation degree of surfactant 2} + \ldots) / (x1 + x2 + \ldots)$$

wherein x1, x2, ... are the number of moles of each alkyl (or alkoxy) sulphate anionic surfactant of the mixture and alkoxylation degree is the number of alkoxy groups in each alkyl sulphate anionic surfactant.

The alkyl sulphated anionic surfactant preferably has an average alkyl chain length of from 8 to 18, preferably from 10 to 14, more preferably from 12 to 14, most preferably from 12 to 13 carbon atoms.

The alkyl sulphate anionic surfactant can have a weight average degree of branching of less than 30%, preferably less than 20%, more preferably less than 10%, and most preferably the alkyl chain of the alkyl sulphate anionic surfactant is linear.

The weight average degree of branching for an anionic surfactant mixture can be calculated using the following formula:

$$\text{Weight average degree of branching (\%)} = [(x1 * \text{wt\% branched alcohol 1 in alcohol 1} + x2 * \text{wt\% branched alcohol 2 in alcohol 2} + \ldots) / (x1 + x2 + \ldots)] * 100$$

wherein x1, x2, ... are the weight in grams of each alcohol in the total alcohol mixture of the alcohols which were used as starting material before (alkoxylation and) sulphation to produce the alkyl (alkoxy) sulphate anionic surfactant. In the weight average degree of branching calculation, the weight of the alkyl alcohol used to form the alkyl sulphate anionic surfactant which is not branched is included.

The anionic surfactant can comprise the alkyl isosorbide sulphate and the alkyl sulphate anionic surfactant in a weight ratio of from 1:9 to 2:1 preferably 1:5 to 1:1, more preferably from 1:4 to 1:2. Without wishing to be bound by theory, it is believed that a mixture provides a surfactant packing which balances performance, low temperature stability and robustness against water hardness variations.

Suitable examples of commercially available alkyl sulphate anionic surfactants include, those derived from alcohols sold under the Neodol® brand-name by Shell, or the Lial®, Isalchem®, and Safol® brand-names by Sasol, or some of the natural alcohols produced by The Procter & Gamble Chemicals company.

The surfactant system may comprise further anionic surfactant, including sulfonate such as HLAS, or sulfosuccinate anionic surfactants. However, for further improvements in sudsing, the surfactant system can comprise less than 30%, preferably less than 15%, more preferably less than 10% by weight of the anionic surfactant of further anionic surfactant, and most preferably the surfactant system comprises no further anionic surfactant.

Co-Surfactant:

The composition further comprises a co-surfactant selected from the group consisting of an amphoteric surfactant, a zwitterionic surfactant and mixtures thereof, as part of the surfactant system. The composition preferably comprises from 0.1% to 20%, more preferably from 0.5% to 15% and especially from 2% to 10% by weight of the detergent composition of the co-surfactant.

The surfactant system of the detergent composition of the present invention preferably comprises from 10% to 40%, preferably from 15% to 35%, more preferably from 20% to 30%, by weight of the surfactant system of a co-surfactant.

The co-surfactant is selected from the group consisting of an amphoteric surfactant, a zwitterionic surfactant, and mixtures thereof. The co-surfactant is preferably an amphoteric surfactant, more preferably an amine oxide surfactant.

The amine oxide surfactant can be linear or branched, though linear are preferred. Suitable linear amine oxides are typically water-soluble, and characterized by the formula R1 - N(R2)(R3) O wherein R1 is a C8-18 alkyl, and the R2 and R3 moieties are selected from the group consisting of C1-3 alkyl groups, C1-3 hydroxyalkyl groups, and mixtures thereof. For instance, R2 and R3 can be selected from the group consisting of: methyl, ethyl, propyl, isopropyl, 2-hydroxethyl, 2-hydroxypropyl and 3-hydroxypropyl, and mixtures thereof, though methyl is preferred for one or both of R2 and R3. The linear amine oxide surfactants in particular may include linear C10-C18 alkyl dimethyl amine oxides and linear C8-C12 alkoxy ethyl dihydroxy ethyl amine oxides.

Preferably, the amine oxide surfactant is selected from the group consisting of: alkyl dimethyl amine oxide, alkyl amido propyl dimethyl amine oxide, and mixtures thereof. Alkyl dimethyl amine oxides are preferred, such as C8-18 alkyl dimethyl amine oxides, or C10-16 alkyl dimethyl amine oxides (such as coco dimethyl amine oxide). Suitable alkyl dimethyl amine oxides include C10 alkyl dimethyl amine oxide surfactant, C10-12 alkyl dimethyl amine oxide surfactant, C12-C14 alkyl dimethyl amine oxide surfactant, and mixtures thereof. C12-C14 alkyl dimethyl amine oxide are particularly preferred. Preferably, the alkyl chain of the alkyl dimethyl amine oxide is a linear alkyl chain, preferably a C12-C14 alkyl chain, more preferably a C12-C14 alkyl chain derived from coconut oil or palm kernel oil.

Alternative suitable amine oxide surfactants include mid-branched amine oxide surfactants. As used herein, "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of nl and n2 can be from 10 to 24 carbon atoms, preferably from 12 to 20, and more preferably from 10 to 16. The number of carbon atoms for the one alkyl moiety (n1) is preferably the same or similar to the number of carbon atoms as the one alkyl branch (n2) such that the one alkyl moiety and the one alkyl branch are symmetric. As used herein "symmetric" means that |n1 - n2| is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a C1-3 alkyl, more preferably both are selected as C1 alkyl.

Alternatively, the amine oxide surfactant can be a mixture of amine oxides comprising a mixture of low-cut amine oxide and mid-cut amine oxide. The amine oxide of the composition of the invention can then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls and mixtures thereof; and
b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl, with preferably both R1 and R2 being methyl. In the mid-cut amine oxide of formula R4R5R6AO, R4 and R5 are preferably both methyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Limiting the amount of amine oxides of formula R7R8R9AO improves both physical stability and suds mileage.

Suitable zwitterionic surfactants include betaine surfactants. Such betaine surfactants includes alkyl betaines, alkylamidobetaine, amidazoliniumbetaine, sulphobetaine (INCI Sultaines) as well as the Phosphobetaine, and preferably meets formula (I):

$$R^1\text{-}[CO\text{-}X(CH_2)_n]_x\text{-}N^+(R^2)(R_3)\text{-}(CH_2)_m\text{-}[CH(OH)\text{-}CH_2]_y\text{-}Y^-$$

wherein in formula (I),

R1 is selected from the group consisting of: a saturated or unsaturated C6-22 alkyl residue, preferably C8-18 alkyl residue, more preferably a saturated C10-16 alkyl residue, most preferably a saturated C12-14 alkyl residue;
X is selected from the group consisting of: NH, NR4 wherein R4 is a C1-4 alkyl residue, O, and S,
n is an integer from 1 to 10, preferably 2 to 5, more preferably 3,
x is 0 or 1, preferably 1,

R2 and R3 are independently selected from the group consisting of: a C1-4 alkyl residue, hydroxy substituted such as a hydroxyethyl, and mixtures thereof, preferably both R2 and R3 are methyl, m is an integer from 1 to 4, preferably 1, 2 or 3,
y is 0 or 1, and
Y is selected from the group consisting of: COO, SO3, OPO(OR5)O or P(O)(OR5)O, wherein R5 is H or a C1-4 alkyl residue.

Preferred betaines are the alkyl betaines of formula (IIa), the alkyl amido propyl betaine of formula (IIb), the sulphobetaines of formula (IIc) and the amido sulphobetaine of formula (IId):

$R^1\text{-}N(CH_3)_2\text{-}CH_2COO^-$ (IIa)

$R^1\text{-}CO\text{-}NH\text{-}(CH_2)_3\text{-}N^+(CH_3)_2\text{-}CH_2COO^-$ (IIb)

$R^1\text{-}N^+(CH_3)_2\text{-}CH_2CH(OH)CH_2SO_3^-$ (IIc)

$R^1\text{-}CO\text{-}NH\text{-}(CH_2)_3\text{-}N^+(CH_3)_2\text{-}CH_2CH(OH)CH_2SO_3^-$ (IId)

in which R1 has the same meaning as in formula (I). Particularly preferred are the carbobetaines [i.e. wherein $Y^-$=COO- in formula (I)] of formulae (IIa) and (IIb), more preferred are the alkylamidobetaine of formula (IIb).
Suitable betaines can be selected from the group consisting or [designated in accordance with INCI]: capryl/capramidopropyl betaine, cetyl betaine, cetyl amidopropyl betaine, cocamidoethyl betaine, cocamidopropyl betaine, cocobetaines, decyl betaine, decyl amidopropyl betaine, hydrogenated tallow betaine / amidopropyl betaine, isostearamidopropyl betaine, lauramidopropyl betaine, lauryl betaine, myristyl amidopropyl betaine, myristyl betaine, oleamidopropyl betaine, oleyl betaine, palmamidopropyl betaine, palmitamidopropyl betaine, palm-kernelamidopropyl betaine, stearamidopropyl betaine, stearyl betaine, tallowamidopropyl betaine, tallow betaine, undecylenamidopropyl betaine, undecyl betaine, and mixtures thereof. Preferred betaines are selected from the group consisting of: cocamidopropyl betaine, cocobetaines, lauramidopropyl betaine, lauryl betaine, myristyl amidopropyl betaine, myristyl betaine, and mixtures thereof. Cocamidopropyl betaine is particularly preferred.

Nonionic Surfactant:

The surfactant system can further comprise a nonionic surfactant, preferably a nonionic surfactant selected from the group consisting of: alkyl alkoxylated nonionic surfactants, alkylpolyglucosides, and mixtures thereof, more preferably selected from alkylpolyglucosides.
The surfactant system can comprise from 1% to 25%, preferably from 1.25% to 20%, more preferably from 1.5% to 15%, most preferably from 1.5% to 5%, by weight of the surfactant system, of the non-ionic surfactant.
The alkoxylated non-ionic surfactant can be a linear or branched, primary or secondary alkyl alkoxylated non-ionic surfactant, preferably an alkyl ethoxylated non-ionic surfactant, preferably comprising on average from 9 to 15, preferably from 10 to 14 carbon atoms in its alkyl chain and on average from 5 to 12, preferably from 6 to 10, most preferably from 7 to 8, units of ethylene oxide per mole of alcohol.
The compositions of the present invention can comprise alkyl polyglucoside ("APG") surfactant. The addition of alkyl polyglucoside surfactants have been found to improve sudsing beyond that of comparative nonionic surfactants such as alkyl ethoxylated surfactants.
Preferably the alkyl polyglucoside surfactant is a C8-C16 alkyl polyglucoside surfactant, preferably a C8-C14 alkyl polyglucoside surfactant. The alkyl polyglucoside preferably has an average degree of polymerization of between 0.1 and 3, more preferably between 0.5 and 2.5, even more preferably between 1 and 2. Most preferably, the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6.
C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation).

Amphiphilic alkoxylated polyalkyleneimine:

The composition of the present invention may further comprise from about 0.05% to about 2%, preferably from about 0.07% to about 1% by weight of the total composition of an amphiphilic polymer. Suitable amphiphilic polymers

can be selected from the group consisting of: amphiphilic alkoxylated polyalkyleneimine and mixtures thereof. The amphiphilic alkoxylated polyalkyleneimine polymer has been found to reduce gel formation on the hard surfaces to be cleaned when the liquid composition is added directly to a cleaning implement (such as a sponge) before cleaning and consequently brought in contact with heavily greased surfaces, especially when the cleaning implement comprises a low amount to nil water such as when light pre-wetted sponges are used.

Preferably, the amphiphilic alkoxylated polyalkyleneimine is an alkoxylated polyethyleneimine polymer comprising a polyethyleneimine backbone having a weight average molecular weight range of from 100 to 5,000, preferably from 400 to 2,000, more preferably from 400 to 1,000 Daltons. The polyethyleneimine backbone comprises the following modifications:

(i) one or two alkoxylation modifications per nitrogen atom, dependent on whether the modification occurs at an internal nitrogen atom or at an terminal nitrogen atom, in the polyethyleneimine backbone, the alkoxylation modification consisting of the replacement of a hydrogen atom on by a polyalkoxylene chain having an average of about 1 to about 50 alkoxy moieties per modification, wherein the terminal alkoxy moiety of the alkoxylation modification is capped with hydrogen, a C1-C4 alkyl or mixtures thereof;
(ii) a substitution of one C1-C4 alkyl moiety and one or two alkoxylation modifications per nitrogen atom, dependent on whether the substitution occurs at a internal nitrogen atom or at an terminal nitrogen atom, in the polyethyleneimine backbone, the alkoxylation modification consisting of the replacement of a hydrogen atom by a polyalkoxylene chain having an average of about 1 to about 50 alkoxy moieties per modification wherein the terminal alkoxy moiety is capped with hydrogen, a C1-C4 alkyl or mixtures thereof; or
(iii) a combination thereof.

For example, but not limited to, below is shown possible modifications to terminal nitrogen atoms in the polyethyleneimine backbone where R represents an ethylene spacer and E represents a C1-C4 alkyl moiety and X- represents a suitable water soluble counterion:

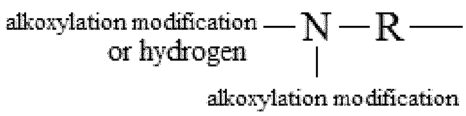

or

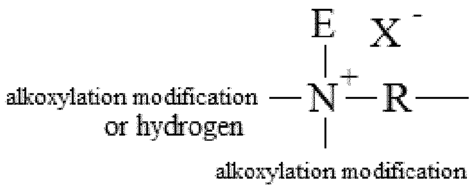

Also, for example, but not limited to, below is shown possible modifications to internal nitrogen atoms in the polyethyleneimine backbone where R represents an ethylene spacer and E represents a C1-C4 alkyl moiety and X- represents a suitable water soluble counterion:

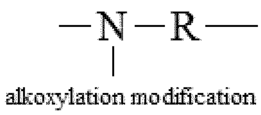

or

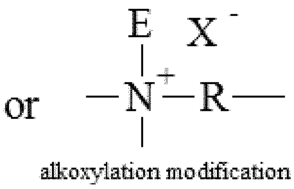

The alkoxylation modification of the polyethyleneimine backbone consists of the replacement of a hydrogen atom by a polyalkoxylene chain having an average of about 1 to about 50 alkoxy moieties, preferably from about 20 to about 45 alkoxy moieties, most preferably from about 30 to about 45 alkoxy moieties. The alkoxy moieties are selected from ethoxy (EO), propoxy (PO), butoxy (BO), and mixtures thereof. Alkoxy moieties solely comprising ethoxy units are outside the scope of use for the invention though. Preferably, the polyalkoxylene chain is selected from ethoxy/propoxy block moieties. More preferably, the polyalkoxylene chain is ethoxy/propoxy block moieties having an average degree of ethoxylation from about 3 to about 30 and an average degree of propoxylation from about 1 to about 20, more preferably ethoxy/propoxy block moieties having an average degree of ethoxylation from about 20 to about 30 and an average degree of propoxylation from about 10 to about 20.

More preferably the ethoxy/propoxy block moieties have a relative ethoxy to propoxy unit ratio between 3 to 1 and 1 to 1, preferably between 2 to 1 and 1 to 1. Most preferably the polyalkoxylene chain is the ethoxy/propoxy block moieties wherein the propoxy moiety block is the terminal alkoxy moiety block.

The modification may result in permanent quaternization of the polyethyleneimine backbone nitrogen atoms. The degree of permanent quaternization may be from 0% to about 30% of the polyethyleneimine backbone nitrogen

atoms. It is preferred to have less than 30% of the polyethyleneimine backbone nitrogen atoms permanently quaternized. Most preferably the degree of quaternization is about 0%.

A preferred amphiphilic alkoxylated polyethyleneimine polymner has the general structure of formula (II):

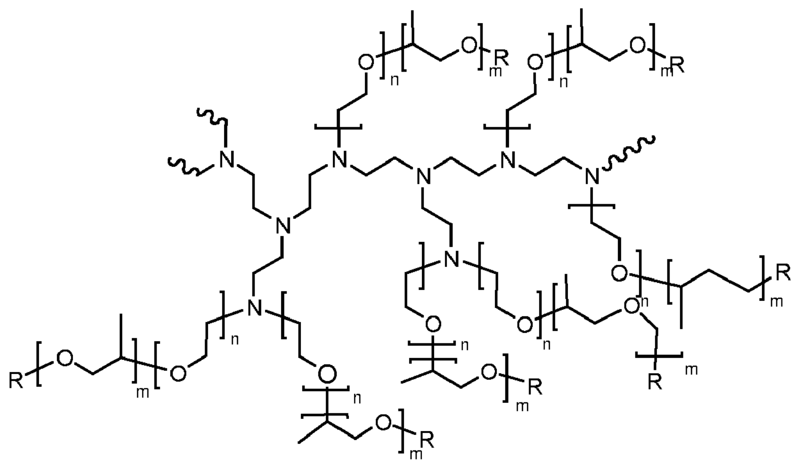

(II)

wherein the polyethyleneimine backbone has a weight average molecular weight of about 600, n of formula (II) has an average of about 10, m of formula (II) has an average of about 7 and R of formula (II) is selected from hydrogen, a C1-C4 alkyl and mixtures thereof, preferably hydrogen. The degree of permanent quaternization of formula (II) may be from 0% to about 22% of the polyethyleneimine backbone nitrogen atoms. The molecular weight of this amphiphilic alkoxylated polyethyleneimine polymer preferably is between 10,000 and 15,000 Da.

More preferably, the amphiphilic alkoxylated polyethyleneimine polymer has the general structure of formula (II) but wherein the polyethyleneimine backbone has a weight average molecular weight of about 600 Da, n of Formula (II) has an average of about 24, m of Formula (II) has an average of about 16 and R of Formula (II) is selected from hydrogen, a C1-C4 alkyl and mixtures thereof, preferably hydrogen. The degree of permanent quaternization of Formula (II) may be from 0% to about 22% of the polyethyleneimine backbone nitrogen atoms, and is preferably 0%. The molecular weight of this amphiphilic alkoxylated polyethyleneimine polymer preferably is between 25,000 and 30,000, most preferably 28,000 Da.

The amphiphilic alkoxylated polyethyleneimine polymers can be made by the methods described in more detail in PCT Publication No. WO 2007/135645.

Cyclic Polyamine

The composition can comprise a cyclic polyamine having amine functionalities that helps cleaning. The composition of the invention preferably comprises from about 0.1% to about 3%, more preferably from about 0.2% to about 2%, and especially from about 0.5% to about 1%, by weight of the composition, of the cyclic polyamine.

The amine can be subjected to protonation depending on the pH of the cleaning medium in which it is used. Preferred cyclic polyamines have the following Formula (III):

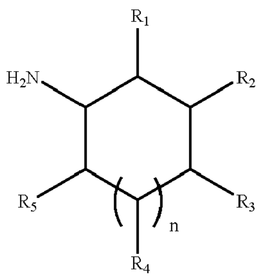

(III)

wherein $R_1$, $R_2$, $R_3$, $R_4$ and $R_5$ are independently selected from the group consisting of NH2, -H, linear or branched alkyl having from about 1 to about 10 carbon atoms, and linear or branched alkenyl having from about 1 to about 10 carbon atoms, n is from about 1 to about 3, preferably n is 1, and wherein at least one of the Rs is NH2 and the remaining "Rs"

are independently selected from the group consisting of NH2, -H, linear or branched alkyl having about 1 to about 10 carbon atoms, and linear or branched alkenyl having from about 1 to about 10 carbon atoms. Preferably, the cyclic polyamine is a diamine, wherein n is 1, $R_2$ is NH2, and at least one of $R_1$, $R_3$, $R_4$ and $R_5$ is CH3 and the remaining Rs are H.

The cyclic polyamine has at least two primary amine functionalities. The primary amines can be in any position in the cyclic amine but it has been found that in terms of grease cleaning, better performance is obtained when the primary amines are in positions 1,3. It has also been found that cyclic amines in which one of the substituents is -CH3 and the rest are H provided for improved grease cleaning performance.

Accordingly, the most preferred cyclic polyamine for use with the detergent composition of the present invention are cyclic polyamine selected from the group consisting of: 2-methylcyclohexane-1,3-diamine, 4-methylcyclohexane-1,3-diamine and mixtures thereof. These specific cyclic polyamines work to improve suds and grease cleaning profile through-out the dishwashing process when formulated together with the surfactant system of the composition of the present invention.

Additional ingredients:

The composition of the present invention may further comprise at least one active selected from the group consisting of: i) a salt, ii) a hydrotrope, iii) an organic solvent, and mixtures thereof.

Salt:

The composition of the present invention may comprise from about 0.05% to about 2%, preferably from about 0.1% to about 1.5%, or more preferably from about 0.5% to about 1%, by weight of the total composition of a salt, preferably a monovalent or divalent inorganic salt, or a mixture thereof, more preferably selected from: sodium chloride, sodium sulphate, and mixtures thereof. Sodium chloride is most preferred.

Hydrotrope:

The composition of the present invention may comprise from about 0.1% to about 10%, or preferably from about 0.5% to about 10%, or more preferably from about 1% to about 10% by weight of the total composition of a hydrotrope or a mixture thereof, preferably sodium cumene sulfonate.

Organic Solvent:

The composition can comprise from about 0.1% to about 10%, or preferably from about 0.5% to about 10%, or more preferably from about 1% to about 10% by weight of the total composition of an organic solvent. Suitable organic solvents include organic solvents selected from the group consisting of: alcohols, glycols, glycol ethers, and mixtures thereof, preferably alcohols, glycols, and mixtures thereof. Ethanol is the preferred alcohol. Polyalkyleneglycols, especially polypropyleneglycol, is the preferred glycol.

Adjunct Ingredients

The detergent composition may optionally comprise a number of other adjunct ingredients such as builders (preferably citrate), chelants, conditioning polymers, other cleaning polymers, surface modifying polymers, structurants, emollients, humectants, skin rejuvenating actives, enzymes, carboxylic acids, scrubbing particles, perfumes, malodor control agents, pigments, dyes, opacifiers, pearlescent particles, inorganic cations such as alkaline earth metals such as Ca/Mg-ions, antibacterial agents, preservatives, viscosity adjusters (e.g., salt such as NaCl, and other mono-, di- and trivalent salts) and pH adjusters and buffering means (e.g. carboxylic acids such as citric acid, HCl, NaOH, KOH, alkanolamines, carbonates such as sodium carbonates, bicarbonates, sesquicarbonates, and alike).

Method of Washing

The invention is further directed to a method of manually washing dishware with the composition of the present invention. The method comprises the steps of delivering a composition of the present invention to a volume of water to form a wash solution and immersing the dishware in the solution. The dishware is cleaned with the composition in the presence of water. Optionally, the dishware can be rinsed. By "rinsing", it is meant herein contacting the dishware cleaned with the process according to the present invention with substantial quantities of appropriate solvent, typically water. By "substantial quantities", it is meant usually about 1 to about 20 L, or under running water.

The composition herein can be applied in its diluted form. Soiled dishware are contacted with an effective

amount, typically from about 0.5 mL to about 20 mL (per about 25 dishes being treated), preferably from about 3 mL to about 10 mL, of the detergent composition, preferably in liquid form, of the present invention diluted in water. The actual amount of detergent composition used will be based on the judgment of the user, and will typically depend upon factors such as the particular product formulation of the detergent composition, including the concentration of active ingredients in the detergent composition, the number of soiled dishes to be cleaned, the degree of soiling on the dishes, and the like. Generally, from about 0.01 mL to about 150 mL, preferably from about 3 mL to about 40 mL of a detergent composition of the invention is combined with from about 2,000 mL to about 20,000 mL, more typically from about 5,000 mL to about 15,000 mL of water in a sink. The soiled dishware are immersed in the sink containing the diluted detergent compositions then obtained, before contacting the soiled surface of the dishware with a cloth, sponge, or similar cleaning implement. The cloth, sponge, or similar cleaning implement may be immersed in the detergent composition and water mixture prior to being contacted with the dishware, and is typically contacted with the dishware for a period of time ranged from about 1 to about 10 seconds, although the actual time will vary with each application and user. The contacting of cloth, sponge, or similar cleaning implement to the dishware is accompanied by a concurrent scrubbing of the dishware.

Alternatively, the composition herein can be applied in its neat form to the dish to be treated. By "in its neat form", it is meant herein that said composition is applied directly onto the surface to be treated, or onto a cleaning device or implement such as a brush, a sponge, a nonwoven material, or a woven material, without undergoing any significant dilution by the user (immediately) prior to application. "In its neat form", also includes slight dilutions, for instance, arising from the presence of water on the cleaning device, or the addition of water by the consumer to remove the remaining quantities of the composition from a bottle. Therefore, the composition in its neat form includes mixtures having the composition and water at ratios ranging from 50:50 to 100:0, preferably 70:30 to 100:0, more preferably 80:20 to 100:0, even more preferably 90:10 to 100:0 depending on the user habits and the cleaning task.

Such methods of neat application comprise the step of contacting the liquid hand dishwashing detergent composition in its neat form, with the dish. The composition may be poured directly onto the dish from its container. Alternatively, the composition may be applied first to a cleaning device or implement such as a brush, a sponge, a nonwoven material, or a woven material. The cleaning device or implement, and consequently the liquid dishwashing composition in its neat form, is then directly contacted to the surface of each of the soiled dishes, to remove said soiling. The cleaning device or implement is typically contacted with each dish surface for a period of time range from 1 to 10 seconds, although the actual time of application will depend upon factors such as the degree of soiling of the dish. The contacting of said cleaning device or implement to the dish surface is preferably accompanied by concurrent scrubbing. Alternatively, the device or implement may be immersed in the liquid hand dishwashing detergent composition in its neat form, in a small container that can accommodate the cleaning device.

Since the compositions of the present invention provide a more consistent sudsing experience, regardless of the hardness of the water used to make the wash solution, the water used to make the wash liquor can have substantially no hardness present at all, for example, when a water softener is installed, or a hardness of as low as 0.15 mmol/l up to as high as 5 mmol/l expressed as $CaCO3$, typically between 0.5 mmol/l and 3 mmol/l pending on the region.

## TEST METHODS

The following assays set forth must be used in order that the invention described and claimed herein may be more fully understood.

### CMC measurement

The critical micelle concentration (CMC) of the different surfactant mixtures tested has been defined through plotting the conductivity of respective surfactant solutions as a function of surfactant concentration at the desired water temperature and hardness. The point where the conductivity versus surfactant concentration slope changes is defined as the CMC value.

2500 ml of water at the desired water hardness is added to a 3 liter beaker placed on a hotplate to heat up the water to the desired test temperature. A mixing blade of sufficient size and set at sufficient mixing speed is positioned in the centre and at 3 cm from the bottom of the beaker to ensure proper mixing of the detergent solutions and timely conductivity equilibration. A conductivity probe connected to a conductivity meter is submerged about 3 cm from the top of the wash solution (Conductivity meter: WTW Cond 3310 - conductivity portable meter ProfiLine Cond 3310). The surfactant concentration is stepwise increased and the conductivity is measured about 20 seconds after each addition to allow proper surfactant hence conductivity equilibration throughout the solution. For each surfactant mixture, a 15% active stock solution was pre-prepared in demineralized water and 0.5ml of the mixed surfactant stock solution was incrementally added at each addition step until at least 3 extra addition steps after a change in slope has been observed. Two straight lines were fitted towards the starting slope and the changed slope respectively. The surfactant concentration at the intercept of the 2 fitted straight lines is defined as the CMC value of the respective surfactant system.

EXAMPLE

The following examples are provided to further illustrate the present invention and are not to be construed as limitations of the present invention, as many variations of the present invention are possible without departing from its scope.

Reduced water hardness sensitivity

The critical micelle concentration was measured using the method described earlier, at two different water hardness and fixed temperature for two surfactant mixtures according to the invention (Examples 1 and 2) comprising sodium dodecyl isosorbide sulphate (5-isomer), synthesized on lab scale using the synthesis method described in J. Colloid Interface Sci, 2011, vol. 360(2), pp645-), as anionic surfactant, and an amine oxide or betaine based cosurfactant system respectively, as well as for two surfactant mixtures outside the scope of the invention (Examples A and B), comprising alkyl ethoxy sulphate anionic surfactant, commonly used as anionic surfactant within liquid hand dishwashing detergent formulations, single variably replacing the sodium dodecyl isosorbide sulphate anionic surfactant within the examples according to the invention. The anionic surfactant and co-surfactant system were added in a 3 to 1 weight ratio.

The data tabulated below clearly shows the decreased water hardness sensitivity of the surfactant mixtures according to the invention, as evidenced by a lower delta CMC value when cross-comparing the results at the two different water hardnesses.

Materials used:

- Sodium dodecyl isosorbide sulphate (SDIS)
- Sodium C12-13 alkyl ethoxy (0.6) sulphate (degree of branching : 25.76%) (AES)
- C1214 alkyl dimethyl amine oxide (AO)
- Cocoamidopropylbetaine (CAPB)

Table 1 : CMC-values for the surfactant mixtures, measured at a temperature of 35°C and under both soft water condition (0.35 mmol/l expressed as CaCO3) and under hard water condition (2.67 mmol/l expressed as CaCO3).

| **CMC (mmol/l)** | **Example 1 (SDIS - AO in 3:1 ratio)** | **Example 2 (SDIS - CAPB in 3:1 ratio)** | **Example A (AES - AO in 3:1 ratio)** | **Example B (AES - CAPB in 3:1 ratio)** |
|---|---|---|---|---|
| 35°C - 2.67 mmol/l | 0.307 | 0.384 | 0.480 | 0.603 |
| 35°C - 0.35 mmol/l | 0.337 | 0.365 | 0.277 | 0.554 |
| Delta CMC (hard vs soft water) | -0.030 | 0.019 | 0.203 | 0.049 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A liquid detergent composition comprising from 5% to 50% by weight of the total composition of a surfactant system, wherein the surfactant system comprises an anionic surfactant at a level of from 60% to 90% weight of the surfactant system, and a co-surfactant, wherein:

i) the anionic surfactant comprises from 10% to 100% by weight of the anionic surfactant of an alkyl isosorbide sulphate anionic surfactant,
wherein the alkyl isosorbide sulphate anionic surfactant has a structure according to formula (I), or an isomer thereof:

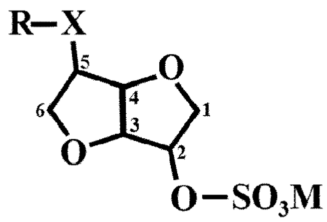

(I)

wherein:

R has an average alkyl chain length of from 8 to 18 carbon atoms;
X is a linking group;
M is a cation,
wherein the alkyl isosorbide sulphate anionic surfactant comprises at least 70% by weight of the alkyl isosorbide sulphate anionic surfactant of the 5-isomer alkyl isosorbide sulphate anionic surfactant, wherein the 5-isomer alkyl isosorbide sulphate anionic surfactant has the alkyl chain R bound via the linking group X to the 5 position in the isosorbide group; and

ii) the co-surfactant is selected from the group consisting of an amphoteric surfactant, a zwitterionic surfactant and mixtures thereof
wherein the pH of the composition is at least 7.0 as measured at 10% dilution in distilled water at 20°C.

**2.** The composition according to claim 1, wherein the liquid detergent composition comprises from 8% to 45%, preferably from 15% to 40%, by weight of the total composition of the surfactant system.

**3.** The composition according to any preceding claim, wherein the composition comprises from 65% to 85%, preferably from 70% to 80% by weight of the surfactant system of anionic surfactant.

**4.** The composition according to any of the preceding claims, wherein the anionic surfactant comprises from 20% to 80%, preferably from 25% to 35% by weight of the anionic surfactant of the alkyl isosorbide sulphate anionic surfactant.

**5.** The composition according to any of the preceding claims, wherein in the alkyl isosorbide sulphate anionic surfactant according to formula (I), or an isomer thereof:

R has an average alkyl chain length of from 10 to 14, preferably from 12 to 14, more preferably from 12 to 13 carbon atoms;
X is a linking group selected from an oxygen atom (-O-), an ester group (-(C=O)-O-), or a mixture thereof, preferably an oxygen atom;
M is a cation counter-ion selected from H, an alkali metal atom, an alkali earth metal ion, an ammonium, an alkylammonium ion, or a mixture thereof, preferably M is an alkali metal ion, more preferably M is Sodium,
wherein the alkyl isosorbide sulphate anionic surfactant comprises at least 80%, preferably at least 85% by weight of the alkyl isosorbide sulphate anionic surfactant of the 5-isomer alkyl isosorbide sulphate anionic surfactant, wherein the 5-isomer alkyl isosorbide sulphate anionic surfactant has the alkyl chain R bound via the linking group X to the 5 position in the isosorbide group.

**6.** The composition according to claim 1, wherein R is linear, preferably naturally derived, more preferably derived from coconut oil, palm kernel oil, and mixtures thereof, most preferably coconut oil.

**7.** The composition according to any of the preceding claims, wherein the anionic surfactant further comprises an alkyl sulphate surfactant, preferably wherein the alkyl sulphate surfactant has a weight average degree of alkoxylation of less than 3, preferably less than 2.0, more preferably less than 1.0, and most preferably wherein the alkyl sulphate surfactant is not alkoxylated.

**8.** The composition according to claim 7, wherein the average alkyl chain length of the alkyl sulphate anionic surfactant is from 8 to 18, preferably from 10 to 14, more preferably from 12 to 14, most preferably from 12 to 13 carbon atoms.

**9.** The composition according to any of claims 7 or 8, wherein the alkyl sulphate anionic surfactant has a weight average degree of branching of less than 30%, preferably less than 20%, more preferably less than 10%, and most preferably the alkyl chain of the alkyl sulphate anionic surfactant is linear.

**10.** The composition according to any of claims 7 to 9, wherein the anionic surfactant comprises the alkyl isosorbide sulphate anionic surfactant and the alkyl sulphate anionic surfactant in a weight ratio of from 1:9 to 2:1 preferably 1:5 to 1:1, more preferably from 1:4 to 1:2.

**11.** The composition according to any of the preceding claims, wherein in the surfactant system, the anionic surfactant and co-surfactant are in a weight ratio of from 1:1 to 9:1, preferably 2:1 to 5:1, more preferably 2.5:1 to 4:1.

**12.** The composition according to any of the preceding claims, wherein the co-surfactant is an amphoteric surfactant, preferably an amine oxide surfactant.

**13.** The composition according to claim 12, wherein the amine oxide surfactant is selected from the group consisting of: alkyl dimethyl amine oxide, alkyl amido propyl dimethyl amine oxide, and mixtures thereof, preferably the amine oxide surfactant is selected from alkyl dimethyl amine oxides selected from the group consisting of: C8-18 alkyl dimethyl amine oxides, more preferably C10-16 alkyl dimethyl amine oxides, more preferably alkyl dimethyl amine oxides selected from the group consisting of: C10 alkyl dimethyl amine oxide surfactant, C10-12 alkyl dimethyl amine oxide surfactant, C12-C14 alkyl dimethyl amine oxide surfactant, and mixtures thereof, most preferably the amine oxide is C12-C14 alkyl dimethyl amine oxide.

**14.** The composition according to any of the preceding claims, wherein the surfactant system further comprises a nonionic surfactant, preferably a nonionic surfactant selected from the group consisting of: alkyl alkoxylated nonionic surfactants, alkylpolyglucosides, and mixtures thereof, more preferably selected from alkylpolyglucosides.

**15.** A method of manually washing dishware comprising the steps of: delivering a composition according to any of claims 1 to 14 to a volume of water to form a wash solution and immersing the dishware in the solution.

Europäisches Patentamt
European Patent Office
Office européen des brevets

# EUROPEAN SEARCH REPORT

Application Number
EP 20 20 1717

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 165 593 A1 (PROCTER & GAMBLE [US]) 10 May 2017 (2017-05-10) * paragraphs [0001], [0006], [0011]; claims 1-15; example A * ----- | 1-15 | INV. C11D1/26 C11D1/94 C11D3/00 C11D11/00 C11D17/00 |
| Y,D | AURELIE LAVERGNE ET AL: "Synthesis and foaming properties of new anionic surfactants based on a renewable building block: Sodium dodecyl isosorbide sulfates", JOURNAL OF COLLOID AND INTERFACE SCIENCE, vol. 360, no. 2, 1 August 2011 (2011-08-01), pages 645-653, XP055200821, ISSN: 0021-9797, DOI: 10.1016/j.jcis.2011.04.110 * page 648 - page 653; tables 1, 2 * ----- | 1-15 | |
| A,D | EP 2 270 017 A1 (COGNIS IP MAN GMBH [DE]) 5 January 2011 (2011-01-05) * paragraphs [0001] - [0003], [0017] - [0019], [0022], [0026], [0033]; figure 1; example 2 * ----- | 1-15 | TECHNICAL FIELDS SEARCHED (IPC) C11D |
| A | WO 2013/041388 A1 (BASF SE [DE]; STOER CLAUDIA [DE] ET AL.) 28 March 2013 (2013-03-28) * page 19 - page 21; claim 38; figure 2; table 4 * ----- | 1-15 | |
| A | WO 99/19440 A1 (PROCTER & GAMBLE [US]; VINSON PHILLIP KYLE [US] ET AL.) 22 April 1999 (1999-04-22) * page 2, paragraph 1 - paragraph 4 * * page 52, line 1 - line 3; claim 1; examples * ----- | 1-15 | |

The present search report has been drawn up for all claims

2

| Place of search | Date of completion of the search | Examiner |
|---|---|---|
| The Hague | 29 March 2021 | Loiselet-Taisne, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 20 1717

This annex lists the patent family members relating to the patent documents cited in the above-mentioned European search report. The members are as contained in the European Patent Office EDP file on
The European Patent Office is in no way liable for these particulars which are merely given for the purpose of information.

29-03-2021

| Patent document cited in search report | Publication date | Patent family member(s) | Publication date |
|---|---|---|---|
| EP 3165593 A1 | 10-05-2017 | AR 106481 A1 | 17-01-2018 |
| | | EP 3165593 A1 | 10-05-2017 |
| | | ES 2718380 T3 | 01-07-2019 |
| | | JP 6711910 B2 | 17-06-2020 |
| | | JP 2018532858 A | 08-11-2018 |
| | | US 2017121637 A1 | 04-05-2017 |
| | | WO 2017074974 A1 | 04-05-2017 |
| EP 2270017 A1 | 05-01-2011 | EP 2270017 A1 | 05-01-2011 |
| | | ES 2428188 T3 | 06-11-2013 |
| | | US 2010324153 A1 | 23-12-2010 |
| WO 2013041388 A1 | 28-03-2013 | BR 112014006482 A2 | 16-05-2017 |
| | | CN 103814033 A | 21-05-2014 |
| | | EP 2758404 A1 | 30-07-2014 |
| | | ES 2719262 T3 | 09-07-2019 |
| | | JP 6396212 B2 | 26-09-2018 |
| | | JP 2014526482 A | 06-10-2014 |
| | | KR 20140072100 A | 12-06-2014 |
| | | US 2014356296 A1 | 04-12-2014 |
| | | WO 2013041388 A1 | 28-03-2013 |
| WO 9919440 A1 | 22-04-1999 | AR 012525 A1 | 18-10-2000 |
| | | AT 288955 T | 15-02-2005 |
| | | AU 9181698 A | 03-05-1999 |
| | | BR 9813065 A | 28-05-2002 |
| | | CN 1281500 A | 24-01-2001 |
| | | CZ 20001355 A3 | 12-09-2001 |
| | | DE 69828989 T2 | 30-03-2006 |
| | | EP 1023426 A1 | 02-08-2000 |
| | | ES 2237848 T3 | 01-08-2005 |
| | | JP 4069968 B2 | 02-04-2008 |
| | | JP 2001520265 A | 30-10-2001 |
| | | US 6281181 B1 | 28-08-2001 |
| | | WO 9919440 A1 | 22-04-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2270017 B1
- EP 2758404 B1
- EP 2343301 B1
- EP 2336281 A1
- EP 3165593 A1
- WO 2013041388 A1
- WO 2007135645 A

### Non-patent literature cited in the description

- **LAVERGNE, A. et al.** Synthesis and foaming properties of new anionic surfactants based on a renewable building block: Sodium dodecyl isosorbide sulfates. *J. Colloid Interface Sci,* 2011, vol. 360 (2), 645-653
- *J. Colloid Interface Sci,* 2011, vol. 360 (2), 645